# DEMANDE DE BREVET EUROPEEN

(11) **EP 0 539 304 A1**
(43) Date de publication de la demande: **28.04.1993**
(21) Numéro de dépôt: 92420371.4
(22) Date de dépôt: 22.10.1992
(51) Int. Cl.: A61K 35/78, A61K 9/16, A61K 47/26

(54) **Procédé de préparation d'extraits adsorbables par voie orale**

(30) Priorité: 24.10.1991 FR 9113355
(71) Demandeur: ETABLISSEMENT RINRONE, Mauren (LI)
(72) Inventeur: Cingotti, Dominique, F-69100 Villeurbanne (FR)
(74) Mandataire: Laurent, Michel

(57) **Abrégé**

Procédé pour la préparation d'un extrait de principes actifs sous forme sèche adsorbable per-os, dans lequel de manière connue :
- tout d'abord, on prépare un extrait de principes actifs dans un milieu solvant spécifique desdits principes actifs ;
- puis, on dépose cet extrait sur des grains d'un support adsorbant ;
- puis, on calibre et on sèche les grains ainsi revêtus ;

**caractérisé** en ce que ensuite, il consiste à adsorber ces grains revêtus dans et sur des microgranules poreuses d'excipients nébulisées, présentant des cavités et des canalicules, à raison de cinq à vingt pourcents (5 à 20 %) en poids du poids desdites microgranules, de manière à remplir partie desdites cavités et desdites canalicules.

Applications envisagées : préparations pharmaceutiques, diététiques ou alimentaires.

## Description

L'invention concerne un nouveau procédé pour la préparation d'un extrait de principes actifs sous forme sèche adsorbable, notamment d'origine végétale, animale ou autres ; elle concerne également des microgranules poreuses adsorbables préparées de la sorte.

Il est bien connu de préparer des extraits de principes actifs, par exemple d'origine naturelle, notamment sous forme de solutions, de macérations ou de teintures-mères. L'administration de ces extraits sous forme extemporanée nécessite des dosages précis, donc difficilement reproductibles.

A ce jour, pour obtenir de tels extraits secs, on fait appel à des procédés mettant en oeuvre un apport calorifique important, tels que nébulisation, rouleaux sécheurs, évaporation sous vide. Si on obtient de la sorte des composés facilement administrables per-os, en revanche, tous ces procédés de préparation, qui font appel à une élévation de température, altèrent les principes actifs des extraits et en modifient la stabilité, notamment du fait de la reprise hygroscopique, du mottage, ce qui entraîne de mauvaises manipulations.

L'invention vise un procédé qui permette d'administrer des principes actifs sous forme solide, plus facile à contrôler et plus facilement reproductible. Elle vise en outre également un procédé qui permette de sélectionner les principes actifs administrés, tout en leur assurant une certaine stabilité dans le temps, sans dégradation thermique.

L'invention pallie ces inconvénients. Elle vise un procédé facile à mettre en oeuvre, permettant d'obtenir des extraits secs adsorbables, riches en produits actifs et stables dans le temps.

Ce procédé pour la préparation d'un extrait de principes actifs sous forme sèche adsorbable per-os, dans lequel, de manière connue :
. tout d'abord, on prépare un extrait de principes actifs dans un milieu solvant spécifique desdits principes actifs ;
. puis, on dépose cet extrait sur des grains d'un support adsorbant ;
. puis, on calibre et on sèche les grains ainsi revêtus,

se caractérise en ce que ensuite, il consiste à adsorber ces grains revêtus dans et sur des microgranules poreuses d'excipients nébulisées, présentant des cavités et des canalicules, à raison de cinq à vingt pourcent (5 à 20 %) en poids du poids desdites microgranules, de manière à remplir partie desdites cavités et desdites canicules.

En d'autres termes, l'invention consiste dans un premier temps, à déposer l'extrait de principes actifs, d'origine végétale, animale ou autres, sur des grains poreux, puis dans un second temps, à faire adsorber ces grains revêtus dans des microgranules poreuses nébulisées dont les cavités et les canalicules sont pour partie essentielle remplies desdits grains de silice revêtus. On obtient de la sorte des microgranules renfermant des principes actifs, stables et facilement adsorbables per-os.

Dans la description et dans les revendications, par "extrait de principes actifs dans un milieu solvant spécifique desdits principes actifs", il faut entendre toute forme de solution contenant des principes actifs, d'origine végétale, animale ou autres, telles que solutions, macérations, teintures-mères. On prépare ces extraits de manière connue, notamment dans un milieu solvant approprié aux principes actifs recherchés. Le plus généralement, on fait appel à de l'éthanol. On peut faire appel à tous extraits végétaux (ou autres) à usage interne (per-os), pharmaceutique ou autres, par un procédé non thermolalile. Le plus généralement, l'extrait contient de 1 à 3 % et de préférence 2 % en produits secs de principes actifs.

En fonction des principes actifs recherchés, on utilise comme milieu solvant soit de l'eau, soit encore mieux un milieu alcoolique, voire hydroalcoolique. Il importe que le milieu solvant alcoolisé soit non toxique et à faible tension de vapeur. En pratique, on fait appel à l'éthanol.

Une fois cet extrait de principes actifs préparé de manière connue, on le dépose, notamment par mouillage, par pulvérisation, .. sur des grains de support adsorbant et compatible, tel que de la silice poreuse colloïdale ou précipitée, voire de cellulose microcristalline, ayant une surface spécifique comprise entre dix et trois cent mètres carrés par gramme. Ces grains poreux sont bien connus et largement utilisés, de sorte qu'il n'est pas utile de les décrire ici en détail. Comme déjà dit, il importe que la surface spécifique de ces grains soit comprise entre dix et trois cent mètres carres par gramme. Comme support adsorbant autre que la silice, on peut également utiliser des celluloses microcristallines, notamment lorsque l'application finale envisagée est à buts homéopathiques.

Le dépôt s'effectue de préférence à température ambiante au mélangeur planétaire, sous léger brassage. On place tout d'abord les grains dans le mélangeur que l'on mouille ensuite par l'extrait, à raison de un volume pour une demie à trois masses. En effet, si le volume d'extrait excède le double de la masse des grains, on obtient un gel difficile à sécher et à manipuler. De même, on a observé que si la quantité de grains excède trois fois le volume de l'extrait, on obtient rapidement une hétérogénéité du mouillage.

Une fois les grains revêtus, on calibre ces grains par passage sur un tamis dont la maille est comprise entre un et deux millimètres, de manière à obtenir des granules parfaitement homogènes. On utilise avantageusement un calibrage mécanique sur tamis oscillant. On sèche ensuite les grains tamisés revêtus sous atmosphère legèrement ventilée, par exemple à 40°C, avec recyclage et récupération du solvant. Une fois le séchage terminé, on recalibre à nouveau sur un tamis plus fin, par exemple de 0,5 millimètre.

Une fois les grains revêtus calibrés, on fait adsorber ces grains imprégnés sur et dans des microgranules poreuses d'excipients nébulisées classiques dans l'industrie pharmaceutique. Ces microgranules nébulisées étant bien connues, il n'y a donc pas lieu de les décrire ici en détail. On utilise avantageusement des microgranules de composés en oses, avantageusement en sorbitol.

Comme on le sait, ces microgranules nébulisées sont obtenues par la technique de pulvérisation-séchage au moyen d'un nébuliseur et présentent une multitude de cavités superficielles ,donc une grande surface spécifique et une multitude de canalicules, c'est-à-dire de petits canaux radiaux qui se dirigent vers l'intérieur des granules, donc une porosité élevée. Ces microgranules ont en pratique une dimension comprise entre 0,1 et un millimètre, de préférence aux alentours de 0,5 millimètre. Il s'ensuit que les grains de silice ou de celluloses microcristallines revêtus des principes actifs, dont les dimensions sont de l'ordre du micromètre, viennent se loger facilement dans les cavités superficielles ainsi que dans les canalicules, et y restent emprisonnées.

Comme microgranules, on utilise avantageusement celles à base de sorbitol, du type de celui qui est commercialisé par MERCK sous la dénomination SORBITOL INSTANT.

Avantageusement, la quantité de grains revêtus représente de 5 à 20 %, et de préférence 10 % du poids de l'excipient nébulisé sous forme de microgranules. On a observé que si la proportion de grains est inférieure à 5 %, on obtenait un mélange hétérogène. En revanche, si cette proportion excède 20 %, on obtient rapidement une saturation avec un déphasage entre la phase fine et la phase grossière, et on augmente de manière inutile le coût de l'opération. Comme déjà dit, on obtient d'excellents résultats avec une proportion voisine de 10 %.

Comme microgranules nébulisées, on fait appel de préférence à des composés en oses qui sont facilement administrables, tels que des composés de sorbitol, manitol, obtenus par nébulisation. Il importe que ce support ait une forte capacité d'adsorption pour retenir les subtances actives et permettent d'obtenir une structure sursaturée en grains revêtus par le remplissage du système réticularisé. Celà permet en outre d'obtenir des formes finales unitaires par compression directe.

Le mélange des microgranules nébulisées et des grains revêtus, s'effectue de manière connue, notamment dans un mélangeur à température ambiante.

On obtient de la sorte des microgranules nébulisées d'excipients adsorbables per-os présentant des cavités et des canalicules, qui se caractérisent en ce que lesdites cavités et lesdites canicules sont remplies de grains, par exemple de silice colloïdale, recouverts d'un extrait actif, végétal notamment.

La manière dont l'invention peut être réalisée et les avantages qui en découlent ressortiront mieux des exemples de réalisation qui suivent.

### Exemple 1:

De manière connue, on prépare une teinture alcoolique de passiflore par macération, pendant quarante-cinq jours à température ambiante, des parties aériennes de la plante dans de l'éthanol à 90°. Après macération, on soutire, on essore, puis on filtre la teinture obtenue. Cette teinture contient environ 0,2 % de flavanoïdes.

On pulvérise ensuite cette teinture à température ambiante sur des grains de silice colloïdale précipitée, commercialisée par DEGUSSA, sous la dénomination FK 320 DS, présentant une surface spécifique de cent soixante-dix (170) m2/ gramme, une grandeur moyenne des particules primaires de dix-huit nanomètres, une valeur de pH de 6,3 et une densité tassée, mais non comprimée de 220. On mélange à raison de un volume de teinture pour une masse de grains de silice. On brasse ces grains mouillés de silice pendant cinq à quinze minutes à température ambiante, de manière à obtenir un mouillage homogène. On obtient ainsi une poudre fine, légèrement mouillée, de couleur vert foncé, rappelant la chlorophylle.

On calibre ensuite les grains revêtus par passage sur un granulateur oscillant, dont la maille est comprise entre 1,5 et deux millimètres (mm). On sèche en couche mince pendant quatre heures environ à 40°C, sous atmosphère ventilée. On recalibre à nouveau sur un granulateur oscillant dont la maille est ramenée à 0,5 mm.

On peut facilement stocker les grains de silice ainsi revêtus.

Dans un mélangeur planétaire, on place quatre-vingt dix volumes de microgranules nébulisées de sorbitol, commercialisées par MERCK A.G. sous la dénomination SORBITOL INSTANT PHARMA 3140, se présentant sous forme de poudre fine blanche, cristalline et inodore, présentant une multitude de cavités superficielles et de canalicules. Ce composé présente une granulométrie moyenne comprise pour 60 à 70 % entre 210 et 500 micromètres et pour 25 à 35 % entre 500 et 850 micromètres. On ajoute ensuite, toujours à température ambiante et au mélangeur dix volumes de grains de silice revêtus ci-dessus. On brasse pour homogénéiser pendant vingt minutes environ.

On obtient ainsi des microgranules de sorbitol, dont les cavités et les canalicules sont remplies de grains de silice recouverts d'extraits secs, porteurs des principes actifs de la passiflore.

Avec ces microgranules, on confectionne des gélules de deux cent cinquante milligrammes, que l'on administre à la demande comme tranquilisant à raison de une à neuf gélules par jour et ce, sans provoquer d'accoutumance. On obtient donc un mélange sursaturé possédant :
. une excellente compressibilité directe ;
. une excellente capacité de liaison avec les principes actifs ;
. une bonne miscibilité avec les principes actifs adsorbés sur la phase intermédiaire avec le support du fait de la faible densité apparente ;
. une bonne stabilité ordonnée, sans risque de déphasage ni "d'écaillage" du support sorbitol.

### Exemple 2:

On répète l'exemple 1 en utilisant non plus une teinture alcoolique de passiflore, mais un extrait polyvégétal destiné à la circulation veineuse dans de l'éthanol à 60°, contenant un mélange en proportions égales d'aubépine, d'olivier et d'ail. On utilise les mêmes proportions et les mêmes constituants qu'à l'exemple 1.

On obtient de la sorte des microgranules de couleur blanche-grise, facile à administrer sous forme de gélules de gélatine alimentaire de deux cent cinquante milligrammes, à raison de deux à six gélules par jour pendant vingt à trente jours.

### Exemple 3:

On répète l'exemple 2 avec des extraits de racines harpagophytum d'orthosiphon, de fumeterre ou de matricaire en proportions égales dans de l'éthanol à 60 %;

On obtient de la sorte comme précédemment, des microgranules adsorbées, faciles à confectionner sous forme de gélules de deux cent cinquantre milligrammes. Ces gélules permettent de traiter efficacement les troubles hépathiques, à raison d'une administration de deux à six gélules par jour pendant vingt à trente jours.

### Exemple 4:

On remplace les grains de silice des exemples précédents par des grains poreux de cellulose microcristalline commercialisés par DEGUSSA, sous la dénomination ELCEMA P 050 ayant les caractéristiques suivantes :
- . tailles des particules: : 40 à 70 micromètres,
- . densité apparente: : 230 g/litre

En opérant comme à l'exemple 1, on mouille deux masses de ces grains, d'un volume de dilution homéopathique (quelque soit la dilution centésimale Hahnemannienne).

En fonction des posologies requises habituellement en homéopathie:
- . tube, granules: : 3 à 5 granules
- . tube globules: : dose unitaire
- . forme gouttes buvables: : proportionnelle à 15 gouttes,

on mélange de 80 à 97 % en poids de microgranules de sorbitol, avec 20 à 13 % de grains de cellulose microcristalline ainsi revêtus.

Le mélange ainsi obtenu peut être dispensé sous des formes variées :
- gélules,
- comprimés avantageusement obtenus par compression directe sans adjuvant,
- sachets, facilement solubles dans l'eau.

### Exemple 5:

On répète l'exemple 1, mais en imprégnant directement les microgranules de sorbitol par la solution hydroalcoolique d'extrait, sans faire appel à l'étape intermédiaire des grains de silice.

On obtient rapidement une prise en masse impossible à homogénéiser, à sécher et à recalibrer.

Le procédé selon l'invention présente de nombreux avantages par rapport à ceux commercialisés à ce jour. On peut citer :
- la possibilité de présenter un extrait liquide actif sous forme sèche, facile à administrer per-os de manière précise et reproductible, ce qui garantit le totum ;
- la simplicité de mise en oeuvre ;
- la facilité d'adsorption orale, puisque le support excipient masque le goût de la teinture, donc amélioration du caractère organoleptique et gustatif du composé administré ;
- la facilité à comprimer ou à administrer ces extraits sous forme de gélules.

De la sorte, ce procédé peut être utilisé avantageusement pour tous les extraits destinés à être administrés per-os sous forme de comprimés, de gélules, de pastilles à sucer, notamment des extraits végétaux, minéraux, animaux ou même des principes actifs, de semi-synthèse ou de synthèse totale, à des fins pharmaceutiques, diététiques, alimentaires ou autres.

## Revendications

**1/** Procédé pour la préparation d'un extrait de principes actifs sous forme sèche adsorbable per-os, dans lequel de manière connue :
- tout d'abord, on prépare un extrait de principes actifs dans un milieu solvant spécifique desdits principes actifs ;
- puis, on dépose cet extrait sur des grains d'un support adsorbant ;
- puis, on calibre et on sèche les grains ainsi revêtus ;
**caractérisé** en ce que ensuite, il consiste à adsorber ces grains revêtus dans et sur des microgranules poreuses d'excipients nébulisées, présentant des cavités et des canalicules, à raison de cinq à vingt pourcents (5 à 20 %) en poids du poids desdites microgranules, de manière à remplir partie desdites cavités et desdites canalicules.

**2/** Procédé selon la revendication 1, caractérisé en ce que les grains du support adsorbant présentent une surface spécifique comprise entre dix et trois cent mètres carrés par gramme.

**3/** Procédé selon la revendication 1, caractérisé en ce que le support adsorbant est choisi dans le groupe comprenant les grains de silice poreux et de cellulose microcristalline.

**4/** Procédé selon la revendication 1, caractérisé en ce que l'on dépose l'extrait de principes actifs sur les grains adsorbants par pulvérisation à température ambiante et sous brassage, à raison d'un volume de la solution d'extrait pour une demie à trois masses de grains adsorbants.

**5/** Procédé selon la revendication 4, caractérisé en ce que l'extrait actif est un extrait végétal ou animal et contient de un à trois pourcents (1 à 3 %) de principes actifs.

**6/** Procédé selon l'une des revendications 1 à 4, caractérisé en ce que l'on calibre les grains adsorbants revêtus d'extraits par passage sur un tamis vibrant, dont les mailles du réseau ont une ouverture de deux millimètres, puis on sèche sous atmosphère ventilée à 40°C, puis à nouveau on repasse sur un tamis plus fin.

**7/** Procédé selon l'une des revendications 1 à 6, caractérisé en ce que les microgranules poreuses sont à base de composés en oses.

**8/** Procédé selon la revendication 7, caractérisé en ce que les microgranules poreuses sont à base de sorbitol.

**9/** Microgranules poreuses nébulisées adsorbables per-os d'excipients pharmaceutiques du type présentant des cavités et des canalicules, caractérisées en ce que partie desdites cavités et desdites canalicules sont remplies de grains adsorbants recouverts en partie d'une couche d'un extrait de principes actifs per-os.

**10/** Microgranules selon la revendication 9, caractérisées en ce que les microgranules sont en sorbitol de 0,5 à un millimètre de diamètre, dont les cavités et les canalicules sont remplies pour partie de grains de silice, revêtus de principes actifs d'extraits d'origine végétale, à raison de dix pourcents (10 %) en poids des grains de silice du poids des microgranules.
